# EUROPEAN PATENT APPLICATION

(11) **EP 4 706 516 A1**
(43) Date of publication of application: **11.03.2026**
(21) Application number: 25200261.3
(22) Date of filing: 04.09.2025
(51) Int. Cl.: A61B 5/0205, A61B 5/145, A61B 5/00

(54) **METHOD OF MEASURING BLOOD GLUCOSE USING LEARNING MODEL**

(30) Priority: 04.09.2024 KR 20240120299
(71) Applicant: I-SENS, INC., Seoul 06646 (KR)
(72) Inventor: LEE, David, 06646 Seoul (KR)
(74) Representative: HGF

(57) **Abstract**

An embodiment may provide a blood glucose measurement method using a learning model, the method including obtaining a biosignal, generating biometric data from the biosignal, training a learning model with training biometric data, so as to output a blood glucose value, obtaining a blood glucose value corresponding to the biometric data from the biometric data via the learning model when training is completed, and providing, to a user, the blood glucose value corresponding to the biometric data.

## Description

### CROSS-REFERENCE TO RELATED PATENT APPLICATION

The present application claims priority under 35 U.S.C. § 119(a) to Korean patent application number 10-2024-0120299 filed on September 4, 2024, in the Korean Intellectual Property Office, the entire disclosure of which is incorporated by reference herein.

### BACKGROUND OF THE INVENTION

### 1. Field

The present disclosure relates to a method of measuring blood glucose, and more specifically, to a technology that obtains a blood glucose value from a biosignal using a learning model and provides the result to a user.

### 2. Description of the Related Art

With recent advancements in medical technology, various medical devices capable of being attached to a user body have been developed and come to the market. Medical devices attached to the user's body may be useful for monitoring bio-information or providing treatment to patients with chronic diseases by attaching them to their skin.

For example, chronic diseases such as diabetes require ongoing management, and medical devices that are attached to the skin and measure blood glucose may be used to monitor blood glucose of diabetic patients. Diabetes shows almost no symptoms in the early stages, but as the disease progresses, characteristic symptoms of diabetes appear, such as polydipsia, polyuria, weight loss, general malaise, itchy skin, and long-lasting wounds on the hands and feet that do not heal. As diabetes progresses, complications such as vision impairment, high blood pressure, kidney disease, stroke, periodontal disease, muscle cramps and neuralgia, and gangrene may appear. To diagnose this type of diabetes and manage it so that it does not progress to complications, systematic blood glucose measurement and treatment need to be performed in parallel.

For people with diabetes and those who do not yet have diabetes but have higher than normal levels of glucose in their blood, many medical device manufacturers offer a variety of blood glucose meters capable of measuring blood glucose.

There are two types of blood glucose meters: one that measures blood glucose one time by drawing blood from a user's fingertip, and one that measures blood glucose continuously by attaching the meter to the user's stomach or arm.

Diabetics typically experience fluctuations in blood glucose levels from hyperglycemia to hypoglycemia. Hypoglycemia is a common emergency, and prolonged hypoglycemia may lead to loss of consciousness or even death. Therefore, prompt detection of hypoglycemic conditions is very important for diabetic patients, but there are limitations in accurately detecting this with blood glucose meters that measure blood glucose intermittently.

Recently, to overcome these limitations, a continuous glucose monitoring system (CGMS) has been developed and is being used, which is inserted into the body and measures blood glucose levels at intervals of several minutes. The CGMS may continuously measure blood glucose by inserting a needle-shaped transcutaneous sensor into areas such as the abdomen and arms where pain is relatively less severe, minimizing the user's pain and discomfort from blood collection.

The CGMS is configured with a sensor transmitter that is inserted into the user's skin to measure blood glucose in the body and transmits the measured blood glucose, and a terminal that outputs the received blood glucose levels.

Here, the terminal may receive biosignals from the sensor transmitter, and may process the signal to generate bio-information (e.g., blood glucose value). In this process, the terminal includes various functional configurations for processing biosignals (e.g., sampling, noise removal, filtering, and calibration), and calculates data through these. When data is computed through multiple functional configurations, the amount of computation becomes very large. In addition, functional configurations for processing biosignals need to be individually set or manipulated by a designer. As the number of functional configurations increases, the designer needs to be directly involved, which increases the workload and thus the time and cost. As artificial intelligence technology has developed recently, it is being applied in various fields. If biosignals are processed using a model that has learned the process of deriving blood glucose values from biosignals, the above-mentioned conventional problems may be resolved.

### SUMMARY OF THE INVENTION

Against this background, one purpose of embodiments of the present disclosure is to provide a technology for a blood glucose measurement method using a learning model to automate a process of deriving a blood glucose value from a biosignal measured from a user.

In addition, another purpose of the present embodiment is to provide a technology for a blood glucose measurement method using a learning model to increase the accuracy of blood glucose value measurement.

In addition, another purpose of embodiments of the present disclosure is to provide a technology for a blood glucose measurement method using a learning model that is trained with various data other than biosignals measured from a user.

In order to achieve the above-mentioned purpose, an embodiment may provide a blood glucose measurement method using a learning model, the method including obtaining a biosignal, generating biometric data from the biosignal, training a learning model with training biometric data, so as to output a blood glucose value, obtaining a blood glucose value corresponding to the biometric data from the biometric data via the learning model when training is completed, and providing, to a user, the blood glucose value corresponding to the biometric data.

In the method, the training biometric data may include past biometric data among the biometric data, and the training of the learning model may train the learning model with the past biometric data.

In the method, the biometric data may include a current value, and the training of the learning model may train the learning model with the current value included in the training biometric data.

In the method, the learning model may include an input layer, a hidden layer, and an output layer that include one or more nodes and are associated with each other based on a weight, and the hidden layer may include a plurality of hidden layers, and two or more active functions may be applied to the plurality of hidden layers.

In the method, the hidden layer may include a first hidden layer to which a first function is applied and a second hidden layer to which a second active function is applied.

In the method, the training of the learning model may include applying the active functions differently to the plurality of hidden layers and determining active functions that satisfy a training criterion for the plurality of hidden layers, and the obtaining of the blood glucose value corresponding to the biometric data may obtain the blood glucose value corresponding to the biometric data via the learning model including the plurality of hidden layer to which the active functions satisfying the training criterion are applied.

In the method, the method may include obtaining blood glucose-related data indicating blood glucose of the user, environmental data including information relating to the user's surrounding environment, and health data including information relating to the user's health status, the training of the learning model may train the learning model with training data including the biometric data, the blood glucose-related data, the environmental data, and the health data, the obtaining of the blood glucose value corresponding to the biometric data may obtain a blood glucose value corresponding to the biometric data, the blood glucose-related data, the environmental data, and the health data, and the providing of the blood glucose value corresponding to the biometric data to the user may provide, to the user, the blood glucose value corresponding to the biometric data, the blood glucose-related data, the environmental data, and the health data.

In the method, the learning model may include an input layer, a hidden layer, and an output layer that include one or more nodes and are associated with each other based on a weight, and two or more different active functions may be selectively applied to the hidden layer depending on the biometric data, the blood glucose-related data, the environmental data, or the health data.

Another embodiment may provide a blood glucose measurement method using a learning model, the method including: obtaining biometric data generated from a biosignal of a user, blood glucose-related data indicating blood glucose of the user, environmental data including information relating to the user's surrounding environment, and health data including information relating to the user's health status; analyzing the biometric data, the blood glucose-related data, the environmental data, and the health data in order to derive a blood glucose characteristic; training a learning model with training data including the biometric data, the blood glucose-related data, the environmental data, and the health data, so as to output the blood glucose value; obtaining the blood glucose value via the learning model when training is completed; correcting the obtained blood glucose value by applying the blood glucose characteristic; and providing the corrected blood glucose value to the user.

In the method, the blood glucose characteristic may include a trend of blood glucose values.

In the method, the learning model may include an input layer, a hidden layer, and an output layer that include one or more nodes and are associated with each other based on a weight, and the hidden layer may include a plurality of hidden layers and two or more active functions may be applied to the plurality of hidden layers.

In the method, the learning model may include an input layer, a hidden layer, and an output layer that include one or more nodes and are associated with each other based on a weight, and two or more different active functions may be selectively applied to the hidden layer depending on the biometric data, the blood glucose-related data, the environmental data, or the health data.

As described above, according to the embodiments, a blood glucose value may be automatically obtained by using a learning model in a blood glucose measurement method.

In addition, according to the embodiments, the accuracy of blood glucose value measurement may be increased by using a learning model that is trained with various data.

In addition, according to the embodiments, a structure of a blood glucose measuring device may be simplified by using a learning model that replaces many functional configurations.

### BRIEF DESCRIPTION OF THE DRAWINGS

FIG. 1 is a diagram for schematically illustrating a blood glucose measurement system according to an embodiment.
FIG. 2 is a diagram for illustrating an applicator for attaching a sensor transmitter to a body according to an embodiment.
FIG. 3 is a diagram for illustrating a process of attaching a sensor transmitter to a body using an applicator according to an embodiment.
FIG. 4 is a configuration diagram of a sensor transmitter according to an embodiment.
FIG. 5 is a configuration diagram of a terminal according to an embodiment.
FIG. 6 is a flowchart illustrating a blood glucose measurement method using a learning model according to an embodiment.
FIG. 7 is an exemplary diagram for illustrating a past biosignal with which a learning model is trained according to an embodiment.
FIG. 8 is a configuration diagram of a learning model according to an embodiment.
FIG. 9 is a configuration diagram of a learning model with a different activation function for each hidden layer according to an embodiment.
FIG. 10 is a configuration diagram of a learning model assigned with a different activation function according to a training result according to an embodiment.
FIG. 11 is a flowchart illustrating a blood glucose measurement method using the learning model of FIG. 10.
FIG. 12 is a configuration diagram of a terminal according to another embodiment.
FIG. 13 is a flowchart illustrating a blood glucose measurement method using a learning model according to another embodiment.

### DETAILED DESCRIPTION

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, it should be understood that terms such as "comprise," "include," or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof.

Terms such as first, second, or the like, are merely identifiers to distinguish identical or corresponding components, and identical or corresponding components are not limited by terms such as first, second, or the like.

Hereinafter, embodiments according to the present disclosure will be described in detail with reference to the attached drawings. In describing with reference to the attached drawings, identical or corresponding components are assigned the same drawing numbers, and redundant descriptions thereof will be omitted.

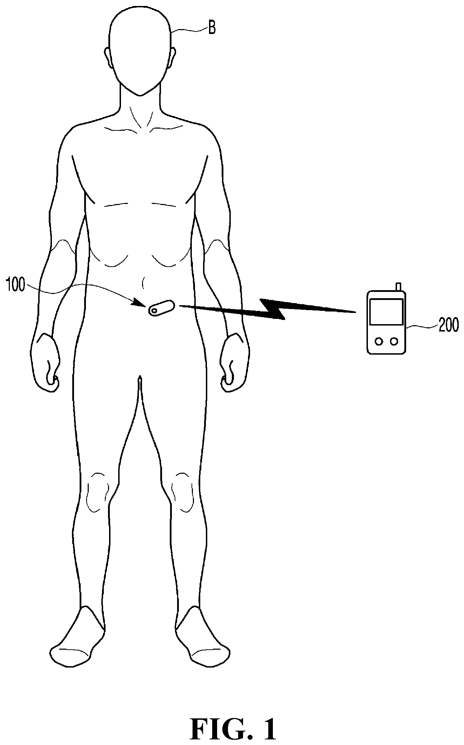

FIG. 1 is a diagram for schematically illustrating a blood glucose measurement system according to an embodiment.

Referring to FIG. 1, a continuous blood glucose measurement system 10 (hereinafter referred to as "system") according to an embodiment may include a sensor transmitter 100 and a terminal 200.

The sensor transmitter 100 is attached to a body (B). When the sensor transmitter 100 is attached to the body (B), one end of a sensor of the sensor transmitter 100 is inserted into skin so that body fluid may be periodically extracted to measure blood glucose.

The terminal 200 may receive a biosignal including blood glucose information from the sensor transmitter 100, may generate blood glucose information from the biosignal, and may output the same to a user. The terminal 200 may include various devices such as a smartphone, a mobile phone, a tablet PC, a desktop, a laptop, or the like, and is not limited thereto and may include a device with a communication interface capable of communicating with the sensor transmitter 100 and on which a program or application may be installed.

The sensor transmitter 100 may transmit a measured biosignal to the terminal 200 at the request of the terminal 200 or periodically at set intervals. For data communication between the sensor transmitter 100 and the terminal 200, the sensor transmitter 100 and the terminal 200 may be connected to each other by wire using a USB cable or the like, or may be connected wirelessly using infrared communication, NFC communication, Bluetooth, or the like.

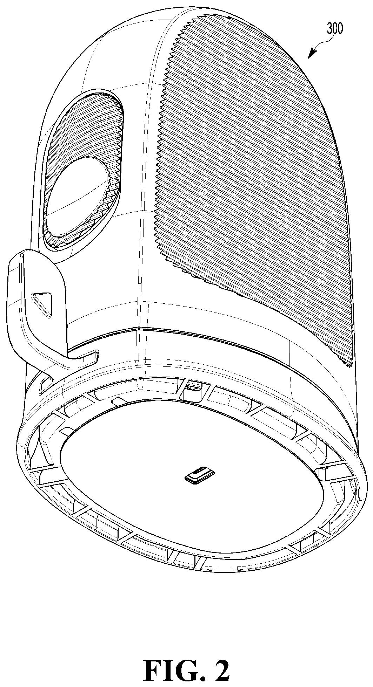

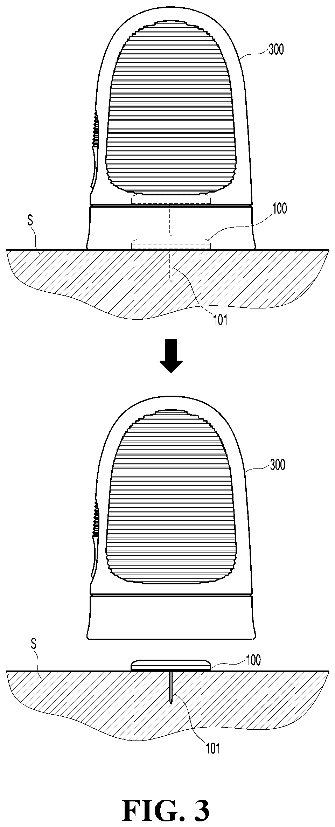

FIG. 2 is a diagram for illustrating an applicator for attaching a sensor transmitter to a body according to an embodiment, and FIG. 3 is a diagram for illustrating a process of attaching a sensor transmitter to a body using an applicator according to an embodiment.

Referring to FIGS. 2 and 3, an applicator 300 according to an embodiment includes the sensor transmitter 100 inside and operates to discharge the sensor transmitter 100 to the outside by a user's operation and attach the same to a predetermined body part of the user. The applicator 300 is provided in a shape with one side open, and the sensor transmitter 100 is installed in the applicator 300 through the open side of the applicator 300.

In the case of attaching the sensor transmitter 100 to a body part using the applicator 300, to insert one end of a sensor provided in the sensor transmitter 100 into skin, the applicator 300 may include a needle (not shown) formed to enclose one end of the sensor inside, a first elastic member (not shown) that pushes the needle and one end of the sensor together into the skin, and a second elastic member (not shown) for pulling out only the needle. Through the configuration of this applicator 300, the needle and one end of the sensor may be simultaneously inserted into the skin by decompression of the first elastic member (not shown) disposed in a compressed state inside the applicator 300. Once the sensor end is inserted into the skin, only the needle is withdrawn by decompressing the compressed second elastic member (not shown). The user may safely and easily attach the sensor transmitter 100 to the skin through the applicator 300.

Looking at the process of attaching the applicator 300 to the body (B) in detail, the open side of the applicator 300 is placed in close contact with a predetermined area of the skin (S) of the body (B) with a protective cap (not shown) removed. When the applicator 300 is operated in this manner while the applicator 300 is in close contact with the skin (S) of the body (B), the sensor transmitter 100 may be attached to the skin (S) while being discharged from the applicator 300. Here, in a lower part of the sensor transmitter 100, one end of a sensor 101 is disposed to be exposed in the sensor transmitter 100, and one end of the sensor 101 may be partially inserted into the skin (S) through a needle provided in the applicator 300. Accordingly, the sensor transmitter 100 may be attached to the skin (S) with one end of the sensor 101 inserted into the skin (S).

Here, an adhesive tape may be provided on a body (B) contact surface of the sensor transmitter 100 so that the sensor transmitter 100 is fixedly attachable to the skin (S) of the body (B). Therefore, when the applicator 300 is separated from the skin (S) of the body (B), the sensor transmitter 100 may be fixedly attached to the skin (S) of the body (B) by the adhesive tape.

Subsequently, when power is applied to the sensor transmitter 100, the sensor transmitter 100 communicates with a terminal, and the sensor transmitter 100 may transmit a biosignal including blood glucose information to the terminal. The sensor transmitter 100 may generate not only blood glucose information but also various pieces of bio-information. In the following description, measuring blood glucose information will be described as an example of measuring bio-information.

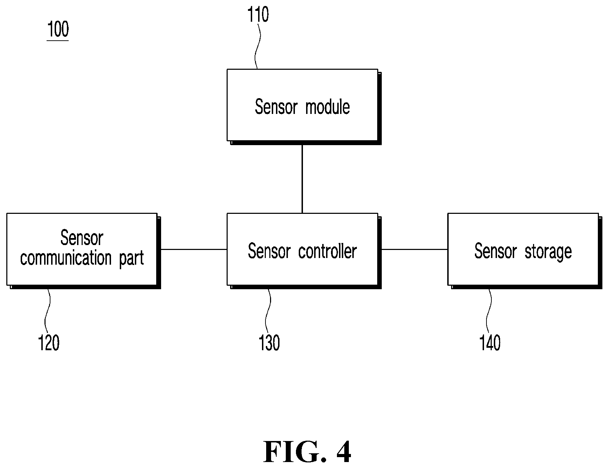

FIG. 4 is a configuration diagram of a sensor transmitter according to an embodiment.

Referring to FIG. 4, the sensor transmitter 100 according to an embodiment may include a sensor module 110, a sensor communication part 120, a sensor controller 130, and a sensor storage 140.

The sensor module 110 may include at least one sensor that is inserted into a body and senses biomass. At least one sensor may measure biomass and generate a biosignal. The biosignal is an analog signal that may include a current value.

The sensor communication part 120 may exchange data or information with a terminal. For example, the sensor communication part 120 may transmit, to the terminal, a biosignal received from the sensor module 110 or data stored in the sensor storage 140.

The sensor controller 130 may control the overall configuration of the sensor transmitter 100, including the sensor module 110, the sensor storage 140, and the sensor communication part 120. For example, the sensor controller 130 may receive a control signal from the terminal and control the configuration of the sensor transmitter 100 accordingly. Additionally, the sensor controller 130 may process a biosignal. For example, the sensor controller 130 may convert a biosignal into an analog or digital form or perform processing to remove noise as needed.

Data or information may be stored in the sensor storage 140. For example, data associated with biomass measured by the sensor module 110 (e.g., current value of biosignal) or data received from a terminal (e.g., command value of control signal) may be stored in the sensor storage 140.

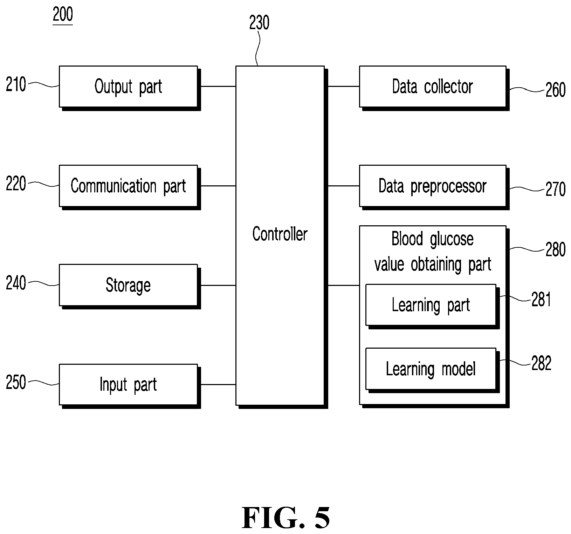

FIG. 5 is a configuration diagram of a terminal according to an embodiment.

Referring to FIG. 5, a terminal 200 according to an embodiment may include an output part 210, a communication part 220, a controller 230, a storage 240, and an input part 250.

The output part 210 may output bio-information (e.g., blood glucose information) included in a biosignal so that a user may check the bio-information. For example, the output part 210 may display blood glucose information as a numerical value or a graph processed from the numerical value.

The communication part 220 may communicate with a sensor communication part of a sensor transmitter and may exchange data or information. For example, the communication part 220 may receive a biosignal including information (i.e., bio-information) associated with biomass measured by the sensor transmitter. Here, the communication part 220 may receive a biosignal that has been primarily processed by the sensor transmitter. Preferably, the processed biosignal may include discrete data (discontinuous data) in which a current value, which is an analog signal, is converted into digital data. When the current value is sampled every cycle, digital discrete data may be generated. Alternatively, the communication part 220 may transmit a control signal to the sensor transmitter to control the sensor transmitter.

Data or information may be stored in the storage 240. For example, data (e.g., bio-information) received from the sensor transmitter may be stored in the storage 240. Here, the bio-information may include blood glucose information and may be in the form of a current value. Alternatively, data input by the user or environment setting data for setting the operating environment of a terminal may be stored in the storage 240.

The input part 250 may receive commands or data from the user. For example, the input part 250 may receive a command for the user to operate the terminal 200 or data (e.g., reference blood glucose value) for obtaining blood glucose information (e.g., blood glucose value). The reference blood glucose value may be used in a calibration process performed when the terminal 200 generates blood glucose information (e.g., blood glucose value). In this case, the user may input a blood glucose value measured by another blood glucose meter into the input part 250 as a reference blood glucose value.

In the following description, blood glucose information is one of the bio-information indicating the user's blood glucose, and may have a current value when measured on the user's skin by the sensor transmitter 100. When blood glucose information is processed in the sensor transmitter 100 or terminal 200, a blood glucose value may be obtained as a predetermined numerical value. In other words, if blood glucose information with a current value goes through data processing, it may be converted into blood glucose information with a blood glucose value.

The controller 230 may include at least one processor that executes a program for measuring blood glucose using a learning model and at least one memory in which the program is stored. The memory and processor included in the controller 230 may be integrated into a single chip or may be physically separated.

Memory may be implemented as non-volatile memory device such as read only memory (ROM), programmable ROM (PROM), erasable programmable ROM (EPROM), electrically erasable programmable ROM (EEPROM) and flash memory, or volatile memory device such as random access memory (RAM) to store various programs, data and/or information.

Additionally, the controller 230 may generate a blood glucose value, which is digitized blood glucose information, from bio-information. To this end, the controller 230 may obtain bio-information in the form of a current value from the sensor transmitter and may preprocess and/or process the current value of the bio-information. The controller 230 may obtain sensitivity first and then generate a blood glucose value based on the sensitivity.

The controller 230 may determine the sensitivity based on a current value and a reference blood glucose value input from the outside, and the sensitivity may be determined based on BGr/C. Here, C refers to a current value at the point in time when sensitivity is determined based on a reference blood glucose value, that is at the point in time when the sensitivity is corrected, and BGr refers to a reference blood glucose value, which is a blood glucose value obtained from a blood glucose monitoring system (BGMS), and does not necessarily need to be obtained at the point in time when the sensitivity is corrected. After the sensitivity is obtained as described above, the controller 230 may multiply the current value and the sensitivity to generate a blood glucose value corresponding to the current value. The controller 230 may provide this new blood glucose value to the user through the output part 240.

The controller 230 may control the terminal 200 to measure blood glucose using a learning model. The controller 230 may control the terminal 200 so that the learning model trained with biometric data generated from biosignals outputs blood glucose information (e.g., blood glucose value) from the biometric data. In order to obtain a blood glucose value through the learning model, the terminal 200 may include a data collector 260, a data preprocessor 270, and a blood glucose value obtaining part 280, and the blood glucose value obtaining part 280 may include a learning part 281 and a learning model 282. The controller 230 may control the data collector 260, the data preprocessor 270, and the blood glucose value obtaining part 280 that implement a blood glucose measurement method through learning. In the following description, description will be provided focusing on the data collector 260, the data preprocessor 270, and the blood glucose value obtaining part 280.

The data collector 260 may collect data for training. The data collector 260 may collect biometric data generated from biosignals. The controller 230 may obtain a biosignal from a sensor transmitter through the communication part 220 and perform signal processing on the biosignal to generate biometric data. For example, in the biosignal, the user's biomass is represented as a current value, and the controller 230 may perform digital signal processing such as noise removal, filtering, sampling, quantization, and encoding on an analog biosignal. And the controller 230 may average digital biosignals. In this way, the controller 230 may generate biometric data. The data collector 260 may acquire the biometric data generated in this way, and the biometric data may be used for training the learning model 282.

In addition, the data collector 260 may collect data from an external device. In addition to biometric data associated with biomass directly sensed from the user, the data collector 260 may obtain blood glucose-related data indicating the user's blood glucose, environmental data including information relating to the user's surrounding environment, and health data including information relating to the user's health status. The terminal 200 may be connected to an external device through the communication part 220 using various wired and wireless communication protocols and may exchange data. The terminal 200 may receive various data including blood glucose-related data, environmental data, and health data from a cloud, a server, or a mobile device. Alternatively, the terminal 200 may receive data through the input part 250. In addition to the biometric data of the sensor transmitter 100 of FIG. 4, the data collector 260 may collect various data including blood glucose-related data, environmental data, and health data through the communication part 220 or input part 250.

The blood glucose-related data may include any direct or indirect data that indicates or suggests the user's blood glucose. The blood glucose-related data does not refer to data such as a biosignal directly collected by a sensor transmitter (100 of FIG. 4,) but may refer to data collected by or obtained from an external device. For example, the blood glucose-related data may include blood glucose values measured by another continuous glucose monitoring system (CGMS) or blood glucose monitoring system (BGMS). The blood glucose-related data may include underlying data that may be used to infer the user's blood glucose. For example, the blood glucose-related data may include a glycated hemoglobin (HbA1C) measurement value. In the case of diabetic patients, the concentration of glucose in the blood increases, so glycated hemoglobin, that is, a glycated hemoglobin level also increases. Therefore, diabetic patients need to regularly check their glycated hemoglobin (HbA1C).

The environmental data may include any data obtained by sensing or measuring the environment surrounding the user. For example, the environmental data may include the user's location or place, temperature, humidity and pressure where the user is, time, acceleration, and the user's activity status (e.g., exercising or eating).

The health data may include any data that directly or indirectly indicates or suggests the user's health status. For example, the health data may include biomass such as a heart rate, a blood pressure, and a body temperature, as well as the frequency and amount of exercise, meals, and sleep as indicators of the user's health.

The data preprocessor 270 may process data for training. The data preprocessor 270 may change data to be suitable for the purpose of training. For example, the data preprocessor 270 may compensate for an outlier or a missing value and process omitted values in the data. The data preprocessor 270 may normalize the data (e.g., min-max normalization or z-score normalization). The data preprocessor 270 may scale data by reducing data deviation.

The blood glucose value obtaining part 280 may obtain a blood glucose value through training. In order to train the learning model 282, the blood glucose value obtaining part 280 may include the learning part 281 and the learning model 282. When training the learning model 282 is completed, the blood glucose value obtaining part 280 may obtain a current blood glucose value using the learning model 282. The obtained blood glucose value may be provided to the user.

Specifically, the learning part 281 may train the learning model 282 so as to output a blood glucose value for data input to the learning model 282. Here, the data input to the learning model, that is, the data with which the learning model 282 is trained, may be biometric data. Alternatively, the data may be blood glucose-related data, environmental data, and/or health data, or the data may include biometric data and blood glucose-related data, environmental data, and/or health data, together. Therefore, the learning part 281 may train the learning model 282 with the biometric data acquired from the sensor transmitter 100 of FIG. 4 and may perform control so that the learning model 282 outputs a blood glucose value corresponding to the biometric data. In addition, the learning part 281 may train the learning model 282 with blood glucose-related data, environmental data, and/or health data and may perform control so that the learning model 282 outputs a blood glucose value corresponding to the blood glucose-related data, environmental data, and/or health data. In addition, the learning part 281 may train the learning model 282 with biometric data, blood glucose-related data, environmental data, and/or health data, and may perform control so that the learning model 282 outputs a blood glucose value corresponding to the biometric data, blood glucose-related data, environmental data, and/or health data.

When the learning part 281 determines that training the learning model 282 has completed, the blood glucose value obtaining part 280 may obtain a blood glucose value through the learning model 282. When the blood glucose value obtaining part 280 receives at least one of biometric data, blood glucose-related data, environmental data, and health data and inputs the same into the learning model 282, the learning model 282 may output a corresponding blood glucose value. The blood glucose value obtaining part 280 may obtain a blood glucose value through the learning model 282 that has completed training. The output part 210 may provide the obtained blood glucose value to the user. Here, when the learning model 282 is trained with biometric data, the blood glucose value obtaining part 280 may input biometric data into the learning model 282 and obtain a blood glucose value corresponding thereto. If the learning model 282 is trained with blood glucose-related data, environmental data, and/or health data, the blood glucose value obtaining part 280 may input these data into the learning model 282 and obtain a blood glucose value corresponding thereto. Therefore, depending on what data the learning model 282 is trained with, data required to obtain a blood glucose value, after training is completed, may be changed.

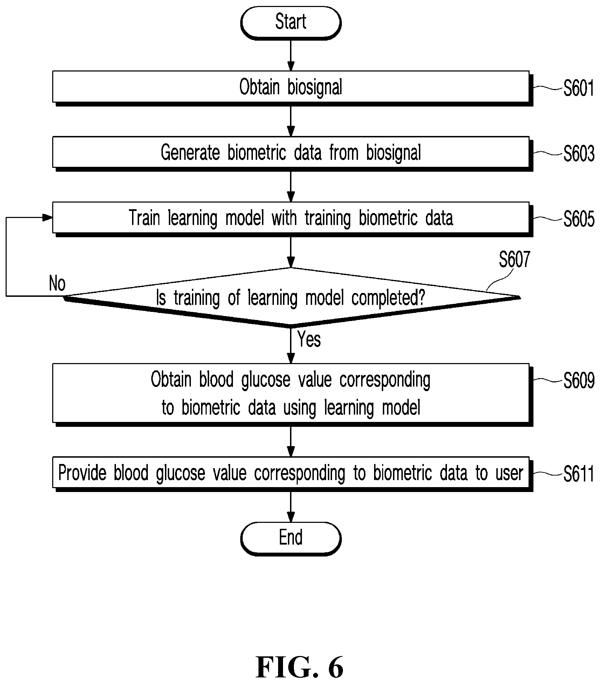

FIG. 6 is a flowchart illustrating a blood glucose measurement method using a learning model according to an embodiment.

FIG. 6 is a flowchart illustrating a blood glucose measurement method of a terminal by using a learning model according to an embodiment. The learning model may be trained with various data and obtain a blood glucose value from them, but in this drawing, it is explained that it is trained with biometric data acquired from a sensor transmitter.

The terminal may receive a biosignal from a sensor transmitter through a communication part in step S601.

The terminal may generate biometric data by performing signal processing on the biosignal through a controller in step S603. The terminal may preprocess biometric data to be suitable for training and may change a portion or the whole of the biometric data into training data. Training data of the biometric data may be referred to as training biometric data.

The terminal may train the learning model with the training biometric data through a learning part in step S605.

The learning part may continue training until the learning model completes training. If the learning part determines that training has not been completed, the learning part may continue to train the learning model in NO of step S607 and step S605. When the learning part determines that training is completed, a blood glucose value obtaining part may obtain a blood glucose value through the learning model. Here, since the learning model has been trained with the biometric data, the blood glucose value obtaining part may obtain a blood glucose value corresponding to new biometric data by inputting the new biometric data into the learning model in YES of step S607 and step S609.

The terminal may provide, to a user, a blood glucose value corresponding to biometric data through an output part in step S611.

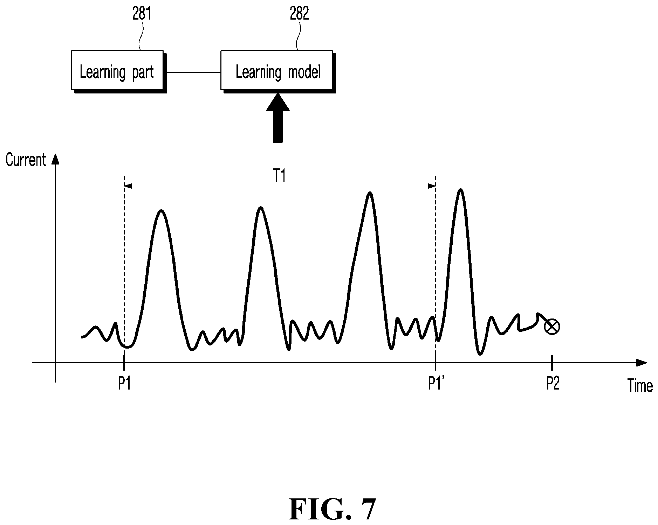

FIG. 7 is an exemplary diagram for illustrating a past biosignal with which a learning model is trained according to an embodiment.

Referring to FIG. 7, an example of training data with which the learning model is trained according to an embodiment may be illustrated. The learning model may be trained with various data (e.g., biometric data, blood glucose-related data, environmental data, or health data) to output a blood glucose value. The learning model may be trained with past data as training data. In particular, the learning model may be trained with past biometric data received from a sensor transmitter, thereby outputting a current blood glucose value, and a terminal may provide the output blood glucose value to a user as the current blood glucose value. Once trained, the learning model may output a blood glucose value in real time and provide the same to the user every time that biometric data is received from the sensor transmitter.

Conventionally, the terminal is required to go through a plurality of functional configurations (e.g., sampling, noise removal, filtering, and calibration) to process biosignals received from the sensor transmitter and output a blood glucose value. However, as in an embodiment, the learning model trained with past biometric data may obtain a blood glucose value in real time without these multiple configurations. Therefore, the terminal's configuration may be simplified, the amount of data computation and computation speed may be increased, and the accuracy of obtaining of the blood glucose value may also be improved.

In the following description associated with the drawing, the past biometric data with which the learning model is trained will be described.

The learning part 281 may train the learning model 282 using past biometric data. The past biometric data may be generated from a biosignal corresponding to a predetermined period of time in the pas among biosignals that the terminal receives from a sensor transmitter. An analog biosignal is converted into biometric data through digital signal processing, and past biometric data may be generated from a past analog biosignal. And the predetermined period of time in the past may be determined based on the amount of training data required for the learning model to accurately obtain the user's blood glucose value. A data preprocessor may generate training biometric data from a biosignal over a predetermined period of time, and the learning part 130 may receive the training biometric data and may train the learning model 282.

For example, when the terminal obtains a blood glucose value through the learning model 282 at a current point in time (P2), the learning model 282 may be trained with past biometric data corresponding to a past period (T1) from time point P1 to time point P1'. The terminal may select past biometric data of one period (T1) as training biometric data and may train the learning model 282. When training is completed, the learning model 282 may receive biometric data with a current value at the current point in time (P2) and may output a corresponding blood glucose value at the current point in time (P2). The terminal may provide, to the user, the blood glucose value at the current point in time (P2).

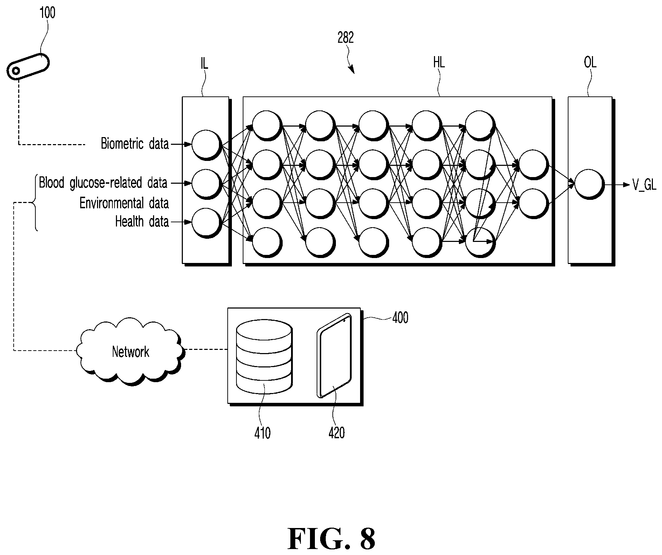

FIG. 8 is a configuration diagram of a learning model according to an embodiment.

Referring to FIG. 8, it is a configuration diagram of the learning model 282 according to an embodiment. The learning model 282 may include various artificial intelligence models, among which may be a machine learning model, including a deep learning model having an artificial neural network. In the following description, the learning model 282 is described as including a deep learning model.

The learning model 282 has an artificial neural network (ANN), and the artificial neural network may have a multilayer perceptron (MLP) structure. When the learning model 282 is configured with a multilayer perceptron structure, the learning model 282 may include an input layer (IL), a hidden layer (HL), and an output layer (OL), and the hidden layer (HL) may include a plurality of layers configured with a plurality of nodes (or neurons). For example, in this drawing, the hidden layer (HL) may include a total of 6 layers, each layer may include 4 nodes, and the last layer may only include 2 nodes. The input layer (IL), hidden layer (HL), and output layer (OL) may be connected to each other based on a weight, and any one node may multiply an output value of a node in a previous layer by a weight, add a bias, and apply an activation function to the product to output a new value.

The learning model 282 having an artificial neural network may receive training data from the input layer (IL) for deep learning, and may perform training. A variety of data is used for training, and these data may be obtained from various sources. A terminal may obtain biometric data from the sensor transmitter 100. The terminal may obtain blood glucose-related data, environmental data, and/or health data from an external device 400. In the external device 400, a server 410 and a mobile device 420 (e.g., another blood glucose meter) may transmit blood glucose-related data, environmental data, and/or health data to the terminal via a wired or wireless network. The learning model 282 may be trained with biometric data, blood glucose-related data, environmental data, and/or health data.

The learning model 282 may be trained to receive training biometric data, training blood glucose-related data, training environmental data, and/or training health data, and output a blood glucose value (V_GL). When the blood glucose value (V_GL) becomes the same as an expected result, the learning part may verify the learning model 282 using verification data. If a training criterion is satisfied, the learning model 282 may be used to obtain a blood glucose value. For example, during a training process, when training biometric data, training blood glucose-related data, training environmental data, and/or training health data are input to the input layer (IL), a blood glucose value (V_GL) may be output based on the weight of each node, bias, and activation function. If the blood glucose value (V_GL) is different from an expected value, the weight may be modified through backpropagation. The process may be repeated until the blood glucose value (V_GL) reaches a desired result using a modified weight. In a verification process, if the learning model 282 is verified based on the verification data and satisfies the training criterion, the learning model 282 may complete training.

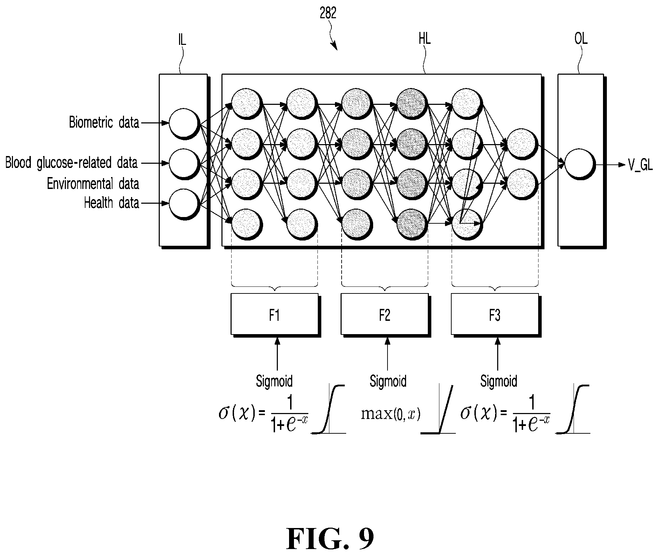

FIG. 9 is a configuration diagram of a learning model with a different activation function for each hidden layer according to an embodiment.

Referring to FIG. 9, a configuration of the learning model 282 having a different activation function for each hidden layer (HL) according to an embodiment may be illustrated. The learning model 282 may include various activation functions to improve training performance. In a multilayer perceptron structure, the hidden layer (HL) may use a single activation function. Each node may calculate an output value of by applying an activation function to a product of a previous output value and a weight. Therefore, in a deep learning model with an artificial neural network, training performance may vary depending on not only appropriate adjustment of a weight but also an activation function applied.

Therefore, the learning model 282 according to an embodiment may apply a different activation function to each hidden layer (HL). The hidden layer (HL) may be configured with a plurality of layers in the multi-perceptron structure, and at least two activation functions may be applied to the plurality of hidden layers (HL). For example, the hidden layer (HL) of the learning model 282 may include six layers (HL1 to HL6). A function provider (F1 to F3) may apply a predetermined activation function to a corresponding hidden layer. A first activation function is applied to the first and second hidden layers (HL1, HL2) among the hidden layers (HL), and the first function provider (F1) may provide the first activation function to the first and second hidden layers (HL1, HL2). A second activation function is applied to the third and fourth hidden layers (HL3, HL4), and the second function provider (F2) may provide the second activation function to the third and fourth hidden layers (HL3, HL4). A third activation function is applied to the fifth and sixth hidden layers (HL5, HL6), and the third function provider (F3) may provide the third activation function to the fifth and sixth hidden layers (HL5, HL6).

Here, the first to third activation functions may all be different from each other, but in some cases, some of them may be the same to improve training performance. As shown in this drawing, the first and second hidden layers (HL1, HL2) and the fifth and sixth hidden layers (HL5, HL6) in the last may have the same sigmoid function, and unlike this, the third and fourth hidden layers (HL3, HL4) in the middle may have an ReLU function.

Applying a different function for each group of the first to sixth hidden layers (HL 1 to HL6) may make up for a weakness caused by applying a single activation function. For example, the sigmoid function is specialized in outputting a probability, but when an input value of the function is very large or very small, an output value approaches 0 (zero) or 1, and a gradient converges to almost 0 (zero), which may cause a problem in which the gradient disappears during backpropagation. As another example, the ReLU function may cause an untrained problem because if an input value is negative, an output value becomes 0 (zero), and many nodes have 0 (zero) values during training. The ReLU function may cause a problem where a gradient may become excessively large when an input value is very large.

On the other hand, some activation functions may have advantages that complement the sigmoid function or the ReLU function. For example, in the ReLU function, a leaky ReLU function may be used to compensate for the problem in that an output value becomes 0 (zero) when an input value is less than 0 (zero). The leaky ReLU function may complement for the weakness of the ReLU function by outputting a constant value other than 0 (zero) as an output value, even if an input value is less than 0 (zero). As another example, a tanh function has an advantage of not causing gradient vanishing because an output value gets closer to 0 (zero) as an input value gets closer to 0 (zero), and converges to 1 or -1 as the input value increases. As another example, a maxout function may output the maximum value among the output values of two ReLU functions. It may compensate for the drawback of the ReLU function in that a gradient is lost.

Therefore, by partially setting activation functions of the hidden layer (HL) to be different, a drawback of one activation function may be compensated for. This may increase training stability and prevent overfitting of training data. For example, in the early layers of the hidden layer (HL), the ReLU function is applied to extract a basic characteristic of data, and in the later layers, the Tanh function is applied to refine the characteristic. As another example, as shown in the drawing, the sigmoid function is applied to the early layers, and to compensate for the sigmoid function's disadvantage of reducing node values, the ReLU function is applied to the middle layers to increase the node values, and the sigmoid function may be applied again to the later layers. This may compensate for the shortcoming of the sigmoid function.

In a state in which different activation functions are applied to the learning model 282, the learning model 282 may be trained to output a blood glucose value (V_GL) from biometric data, blood glucose-related data, environmental data, and/or health data. The learning model 282 in which different activation functions are applied to the hidden layer (HL) in this way may be referred to as a 'hybrid model.'

As described above, the activation function applied to the learning model 282 may be different for each hidden layer (HL), but is not limited thereto and may be selected differently depending on data used for training or a characteristic of the data. The data characteristic may include a data length, a data format (text or image, analog signal or digital signal), or the like.

For example, the learning model 282 may have a different activation function depending on the type of training data. When the learning model 282 is trained with biometric data and obtains a blood glucose value corresponding to the biometric data, an activation function such as the sigmoid function may be applied to the hidden layer (HL). Alternatively, when the learning model 282 is trained with blood glucose-related data, environmental data, and/or health data and obtains a corresponding blood glucose value, an activation function such as the ReLU function may be applied to the hidden layer (HL). Alternatively, when the learning model 282 is trained with biometric data, blood glucose-related data, environmental data, and/or health data together to obtain a blood glucose value, the sigmoid function and the ReLU function may be applied together to the hidden layer (HL). Here, how these multiple activation functions are applied to the hidden layers (HL) may be determined based on training performance as described above. As another example, the learning model 282 may have a different activation function depending on a characteristic of the training data (text or image). When the learning model 282 is trained with text training data and when the model is trained with image training data, the model may use different multiple activation functions.

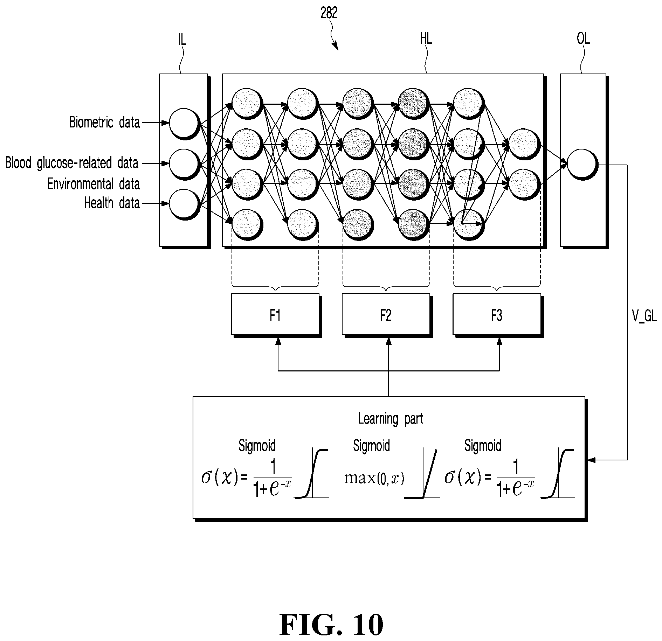

FIG. 10 is a configuration diagram of a learning model assigned with a different activation function according to a training result according to an embodiment.

Referring to FIG. 10, a learning model according to an embodiment may have a different activation function for each hidden layer (HL) based on a training result. The learning part 130 may select, for each hidden layer (HL), an activation function that satisfies a predetermined training criterion or shows the best performance based on the training result. The activation function is not fixed but changes according to a training result during training. This activation function may be referred to as an 'adaptive activation function.' In FIG. 9, a different activation function is determined for each hidden layer (HL) before training and is not changed, whereas in this drawing, a different activation function is provided for each hidden layer (HL), and the activation functions may change according to a training result. If an activation function satisfies the training criterion when applied to several layers of the hidden layer (HL), the activation function may be assigned and applied to the layers.

In the example described above, the first function provider (F1) may provide the first activation function to the first and second hidden layers (HL1, HL2), the second function provider (F2) may provide the second activation function to the third and fourth hidden layers (HL3, HL4), and the third function provider (F3) may provide the third activation function to the fifth and sixth hidden layers (HL5, HL6). The first to third function providers (F1 to F3) may individually receive activation functions from the learning part 281 during training. The learning part 281 may select an activation function to be transmitted to the first to third function providers (F1 to F3) according to a training result. The learning part 281 may include various activation functions such as a sigmoid function, a ReLU function, and a tanh function. The learning part 281 may select at least two activation functions among them according to a training result. The learning part 281 may receive a blood glucose value (V_GL) from an output layer (OL) and may verify a training result. When a predetermined training criterion is satisfied, the learning part 281 may control the first to third function providers (F1 to F3) so as to provide, to the first to sixth hidden layers (HL1 to HL6), the first to third activation functions that were transmitted immediately before, without transmitting new first to third activation functions.

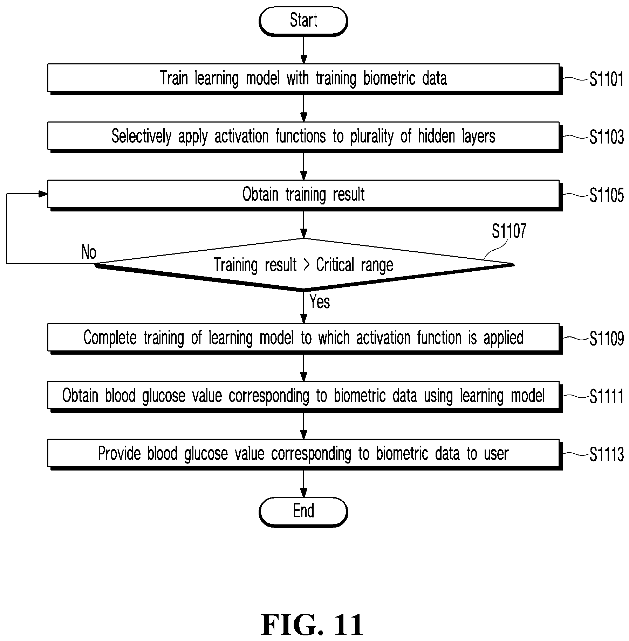

FIG. 11 is a flowchart illustrating a blood glucose measurement method using the learning model of FIG. 10.

Referring to FIG. 11, a method for measuring blood glucose using a learning model in which an activation function is applied to be different depending on a hidden layer according to an embodiment may be illustrated.

A terminal may receive a biosignal from a sensor transmitter and generate biometric data. A learning part of the terminal may train the learning model with training biometric data in step S1101.

The learning model has a deep learning model with an artificial neural network, and the learning part of the terminal may select and apply different activation functions to a plurality of hidden layers in step S1103. The learning part of the terminal may obtain a training result to evaluate a training result of the selected activation functions in step S1105. The training result may include a blood glucose value output from the training data according to the selected activation function.

The learning part of the terminal may determine whether the learning model satisfies a training criterion in step S1107. The learning part of the terminal may determine whether the training result is higher than a critical range (the minimum requirement for completing training as a training criterion). If the training result is not greater than the critical range, the learning part may re-select and apply activation functions for a plurality of hidden layers, and train the learning model in NO of step S1107 and step S1103.

If the training result is greater than the critical range, the learning part may complete training by applying the selected activation function to the learning model in YES of step S1107 and step S1109.

A blood glucose value obtaining part of the terminal may obtain a blood glucose value corresponding to biometric data through the learning model in step S1111. An output part of the terminal may provide the obtained blood glucose value to the user in step S1113.

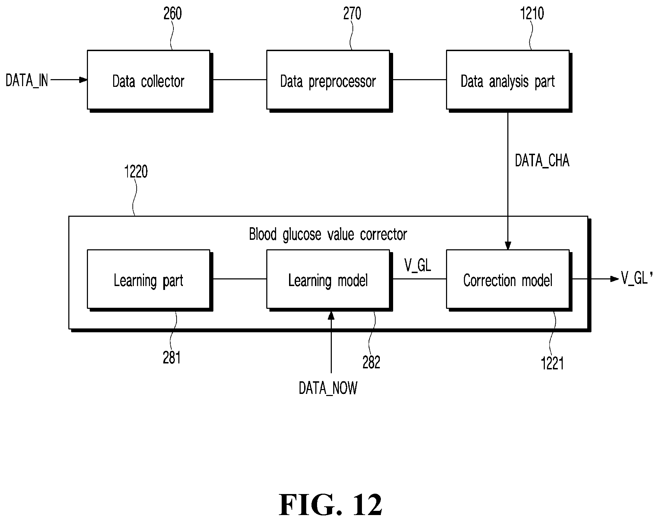

FIG. 12 is a configuration diagram of a terminal according to another embodiment.

Referring to FIG. 12, a terminal according to another embodiment may use a learning model to correct a blood glucose value. The terminal may obtain a blood glucose value through the learning model and then correct the obtained blood glucose value through a correction model. The terminal may not only obtain a blood glucose value in real time through the learning model, but may also improve the accuracy of a blood glucose value by applying a user's blood glucose characteristic derived from data to the obtained blood glucose value.

To this end, the terminal may include a blood glucose value corrector 1220. The blood glucose value corrector 1220 may include the learning model 282 that is trained with data to obtain a blood glucose value, the learning part 281 that trains the learning model 282, and a correction model 1221 that corrects a blood glucose value (V_GL) obtained from the learning model 282. A terminal according to another embodiment has the same configuration as that of an embodiment and some different configurations, and the following description will focus on the differences.

The data collector 260 may collect various data (DATA_IN) that affects a user's blood glucose. Data (DATA_IN) is used for training the learning model 282, and may also be used to derive characteristic data (DATA_CHA) representing the characteristics of the user's blood glucose. Here, the data (DATA_IN) may include biometric data, blood glucose-related data, environmental data, and/or health data.

The data preprocessor 270 may preprocess the data (DATA_IN) including biometric data, blood glucose-related data, environmental data, and/or health data. Preprocessing of data may be performed in the same manner as in an embodiment.

The data analysis part 1210 may derive characteristic data (DATA_CHA) representing the characteristics of the user's blood glucose from the data (DATA_IN). The characteristics of the user's blood glucose may include a trend of blood glucose values, a slope of blood glucose values, and the occurrence of hypoglycemia or hyperglycemia.

The learning part 281 may train the learning model 282 with past data among the data (DATA_IN). The learning model 282 may have an artificial neural network as a deep learning model as in an embodiment. Once training is complete, the learning model 282 may produce a blood glucose value (V_GL). The learning model 282 that has already been trained with the data (DATA_IN) may output a blood glucose value (V_GL) corresponding to current data (DATA_NOW) when the current data (DATA_NOW) is input.

The correction model 1221 may receive characteristic data (DATA_CHA) from the data analysis part 1210. The correction model 1221 may correct a blood glucose value (V_GL) by applying the characteristic data (DATA_CHA) to the blood glucose value (V_GL). Then, the correction model 1221 may finally obtain a corrected blood glucose value (V_GL'), and the terminal may provide the corrected blood glucose value (V_GL') to the user as a current blood glucose value.

For example, if the characteristic data (DATA_CHA) includes a trend of blood glucose values and the trend is rising, the correction model 1221 may adjust the blood glucose values (V_GL) to be slightly higher. If the trend is declining, the correction model 1221 may adjust the blood glucose value (V_GL) to be slightly lower. As another example, if hyperglycemia occurs or occurs frequently, the correction model 1221 may adjust the blood glucose value (V_GL) to be slightly higher. If hypoglycemia occurs or occurs frequently, the correction model 1221 may adjust the blood glucose value (V_GL) to be slightly lower.

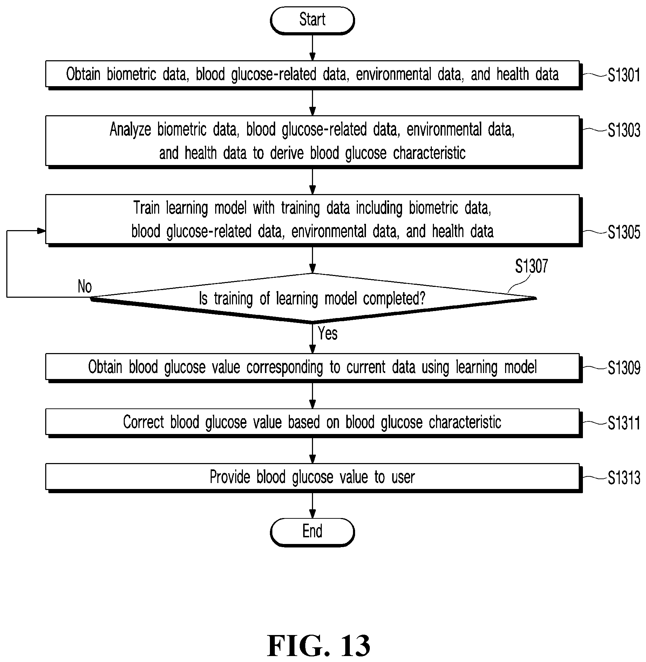

FIG. 13 is a flowchart illustrating a blood glucose measurement method using a learning model according to another embodiment.

Referring to FIG. 13, a blood glucose measurement method using a learning model according to another embodiment may be illustrated. A blood glucose value obtained by the learning model may be corrected by applying characteristic data derived via data analysis.

First, a data collector of a terminal may acquire biometric data, blood glucose-related data, environmental data, and/or health data in step S1301. A data preprocessor may preprocess these data to be suitable for training or analysis.

A data analysis part of the terminal may analyze biometric data, blood glucose-related data, environmental data, and/or health data to derive a characteristic of a user's blood glucose in step S1303.

A learning part of the terminal may train the learning model with training data including biometric data, blood glucose-related data, environmental data, and/or health data in step S1305. The training data may include past data.

When determining that training is not completed, the learning part may continue training in YES of step S1307 and step S1305. When the learning part determines that training is completed, the terminal may use the learning model to obtain a blood glucose value. The learning model may receive current data as input and obtain a blood glucose value corresponding to the current data in step S1309.

In a blood glucose value corrector of the terminal, the correction model 1221 may correct the obtained blood glucose value in step S1311, by receiving characteristic data is received from the data analysis part 1210, and applying the characteristic data to the obtained blood glucose value, thereby outputting a corrected blood glucose value.

The output part of the terminal may provide the corrected blood glucose value as a current blood glucose value (blood glucose value corresponding to current data) to the user in step S1313.

Aspects of the subject matter described herein may be described in the context of computer-executable instructions, such as program modules, running on a computer. Generally, program modules include routines, programs, objects, components, data structures, or the like, and perform specific tasks or specific abstract data types.

Alternatively or additionally, the function described herein may be performed, at least in part, by one or more hardware logic components. By way of example, and not limitation, exemplary types of hardware logic components that may be used include field-programmable gate arrays (FPGAs), program-specific integrated circuits (ASICs), application-specific standard products (ASSPs), system-on-a-chip systems (SOCs), complex programmable logic devices (CPLDs), and the like.

Meanwhile, the disclosed embodiments may be implemented in the form of a recording medium storing programs and/or an instruction executable by a computer. The instruction may be stored in the form of program code, which, when executed by a processor, may generate a program module to perform the operations of the disclosed embodiments. The recording medium may be implemented as a computer-readable recording medium.

The computer-readable recording medium includes all types of recording media that store instructions that may be interpreted by a computer. Examples include read-only memory (ROM), random access memory (RAM), magnetic tape, magnetic disks, flash memory, and optical data storage devices.

Although embodiments of the present disclosure have been described above, those skilled in the art will be able to make various modifications and changes to the present disclosure by adding, changing or deleting components, without departing from the spirit of the present disclosure as set forth in the appended claims, which are included within the scope of rights of the present disclosure.

## Claims

1. A method of measuring blood glucose using a learning model, the method comprising:
obtaining (S601) a biosignal;
generating (S603) biometric data from the biosignal;
training (S605) a learning model with training biometric data, so as to output a blood glucose value;
when training is completed, obtaining (S609) a blood glucose value corresponding to the biometric data from the biometric data via the learning model; and
providing (S611), to a user, the blood glucose value corresponding to the biometric data.

2. The method according to claim 1, wherein the training biometric data includes past biometric data among the biometric data, and
wherein the training (S605) of the learning model comprises training the learning model with the past biometric data.

3. The method according to claim 1, wherein the biometric data includes a current value, and
wherein the training (S605) of the learning model comprises training the learning model with the current value included in the training biometric data.

4. The method according to claim 1, wherein the learning model comprises an input layer, a hidden layer, and an output layer that include one or more nodes and are associated with each other based on a weight, and
wherein the hidden layer comprises a plurality of hidden layers, and two or more active functions are applied to the plurality of hidden layers.

5. The method according to claim 4, wherein the hidden layer comprises a first hidden layer to which a first function is applied and a second hidden layer to which a second active function is applied.

6. The method according to claim 4, wherein the training (S605) of the learning model comprises:
applying the active functions differently to the plurality of hidden layers; and
determining active functions that satisfy a training criterion for the plurality of hidden layers, and
wherein the obtaining (S609) of the blood glucose value corresponding to the biometric data comprises obtaining the blood glucose value corresponding to the biometric data via the learning model including the plurality of hidden layer to which the active functions satisfying the training criterion are applied.

7. The method according to claim 1, further comprising:
Obtaining (S1301) blood glucose-related data indicating blood glucose of the user, environmental data including information relating to the user's surrounding environment, and health data including information relating to the user's health status,
wherein the training (S605) of the learning model comprises training (S1305) the learning model with training data including the biometric data, the blood glucose-related data, the environmental data, and the health data,
wherein the obtaining (S609) of the blood glucose value corresponding to the biometric data comprises obtaining (S1309) a blood glucose value corresponding to the biometric data, the blood glucose-related data, the environmental data, and the health data, and
wherein the providing (S611) of the blood glucose value corresponding to the biometric data to the user comprises providing (S1313), to the user, the blood glucose value corresponding to the biometric data, the blood glucose-related data, the environmental data, and the health data.

8. The method according to claim 7, wherein the learning model comprises an input layer, a hidden layer, and an output layer that include one or more nodes and are associated with each other based on a weight, and
wherein two or more different active functions are selectively applied to the hidden layer depending on the biometric data, the blood glucose-related data, the environmental data, or the health data.

9. A method of measuring blood glucose using a learning model, the method comprising:
obtaining (S1301) biometric data generated from a biosignal of a user, blood glucose-related data indicating blood glucose of the user, environmental data including information relating to the user's surrounding environment, and health data including information relating to the user's health status;
analyzing (S1303) the biometric data, the blood glucose-related data, the environmental data, and the health data in order to derive a blood glucose characteristic;
training (S1305) a learning model with training data including the biometric data, the blood glucose-related data, the environmental data, and the health data, so as to output the blood glucose value;
when training is completed, obtaining (S1309) the blood glucose value via the learning model;
correcting (S1311) the obtained blood glucose value by applying the blood glucose characteristic; and
providing (S1313) the corrected blood glucose value to the user.

10. The method according to claim 9, wherein the blood glucose characteristic includes a trend of blood glucose values.

11. The method according to claim 9, wherein the learning model comprises an input layer, a hidden layer, and an output layer that include one or more nodes and are associated with each other based on a weight, and
wherein the hidden layer comprises a plurality of hidden layers and two or more active functions are applied to the plurality of hidden layers.

12. The method according to claim 9, wherein the learning model comprises an input layer, a hidden layer, and an output layer that include one or more nodes and are associated with each other based on a weight, and
wherein two or more different active functions are selectively applied to the hidden layer depending on the biometric data, the blood glucose-related data, the environmental data, or the health data.
